# EUROPEAN PATENT APPLICATION

(11) **EP 0 558 189 A1**
(43) Date of publication of application: **01.09.1993**
(21) Application number: 93300720.5
(22) Date of filing: 01.02.1993
(51) Int. Cl.: C07D 245/02, C07D 273/00

(54) **A two-step method for preparing cyclic-ureas**

(30) Priority: 19.02.1992 US 837131
(71) Applicant: TEXACO CHEMICAL COMPANY, Houston, Texas 77056 (US)
(72) Inventor: Speranza, George Phillip, Austin, Texas 78757 (US); Champion, Donald Hugh, Pflugerville, Texas 78660 (US)
(74) Representative: Brock, Peter William

(57) **Abstract**

Urea and a diamine can be reacted to form cyclic ureas in a simple two-stage process.

Urea and a diamine are first heated in equimolar amounts at 120 to 140°C, until one mole of ammonia is liberated, and the resulting intermediate is cyclized by heating in a solvent at a temperature from 160 to 200°C.

## Description

This invention relates to cyclic ureas. More particularly this invention relates to an economical two-step method of preparing large ring cyclic urea products from commercially available reactants, which comprises heating a diamine with urea under specifically controlled conditions.

This method provides a very efficient and cost effective means of producing large ring cyclic ureas from commercially available diamines.

The preparation of cyclic ureas is known in the art. Cyclic ureas with 5, 6 and 7 membered rings are not difficult to prepare. They are often synthesized by the reaction of diamines with phosgene, urea or carbon dioxide. Methods for the preparation of cyclic products of higher molecular weight are complicated by the side reaction of linear polycondensation; see G. A. Settepani et al., J. heterocyclic Chem. 10,639 (1973); J. Michus, J. Org. Chem. 25, 2246 (1960); and L. Birkofer et al., Chem. Ber. 93, 2810 (1960).

Large ring cyclic ureas can also be prepared by careful reaction of diisocyanates with water, utilizing a high dilution technique; see S. Ozaki et al., J. Am. Chem. Soc. 79, 4358 (1957). For example, utilizing 900 cm³ of acetone and 900 cm³ of water as reaction medium, 3.2g of octamethylene diisocyanate in 70 cm³ of acetone was added over a 17 hour period in a special Hunsdiecker apparatus. The yield was 18%. 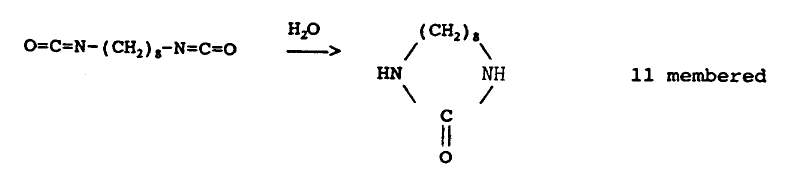
The yields increased to 49% for a 13 membered ring, 41% for a 15 membered ring and 36% for a 17 membered ring.

In Synthesis, 1982, 464, A. V. Bogatsky et al. disclose that macrocyclic thioureas can be obtained by the reaction of diamines with carbon disulphide. The cyclic thioureas react with alkyl halides, in the presence of aqueous sodium hydroxide and catalytic amounts of benzyltriethylammonium chloride, to produce the corresponding N,N'-dialkylureas in good yields. 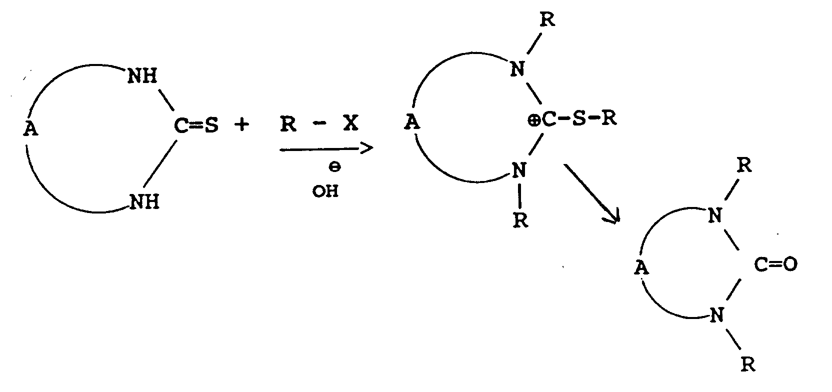
Further, Bogatsky et al. suggest cyclic N,N'-dialkylureas having 5 to 22 carbon atoms could be prepared using this method. The macrocyclic polyoxyethylene ureas are described by this group as a new type of crown ether. Large cyclic rings allow the carbonyl groups to orient themselves into the cavity, which permits coordination of a metal ion with both the ether and the carbonyl oxygen atoms. Ligans such as those having the formulae given below would interact with lithium, sodium and potassium ions (R=alkyl). 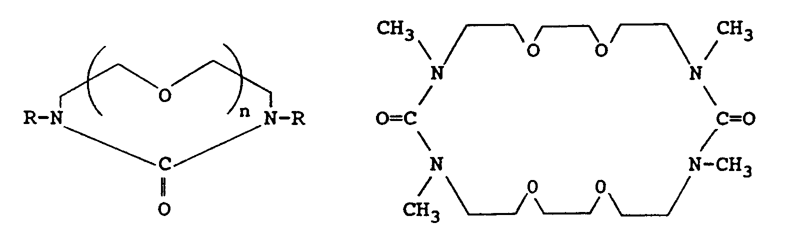
Later, N. G. Lukyanenko et al. reported in Synthesis, 1986, 928 that macrocyclic thioureas may be converted into macrocyclic ureas by another route, which can be represented by the following: 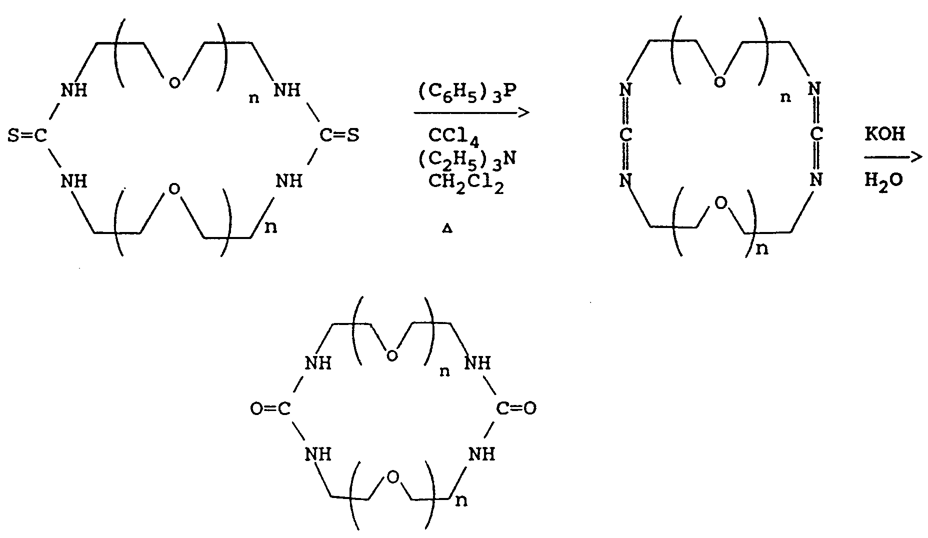
As noted, in a number of the known methods for preparing cyclic ureas, disadvantages include side reactions involving linear polycondensations and the need initially to prepare a diisocyanate derivative of the diamine. Cyclization of the diisocyanate requires very high dilution techniques.

It would represent a substantial advance in the art if cyclic ureas could be prepared from commercially available reactants such as diamines and urea under very controlled reaction conditions without the necessity for high dilution techniques.

This invention, therefore, is concerned with the problem of providing cyclic ureas by a method avoiding high dilution techniques, or an excessive number of stages.

US-A-3763106 discloses a process for preparing water soluble linear polyurea polymers which involves reacting urea with a polyoxyalkylene diamine at a temperature of 140 to 220°C, and then reacting the resulting reaction product with formaldehyde in order to introduce methylol groups.

We have now found that by careful temperature control, it is possible to react urea with diamines to form cyclic ureas.

The present invention provides a method for preparation of cyclic ureas comprising:
(a) heating urea with a diamine whose amino groups are separated by 8 to 22 atoms in a molar ratio of about 1:1, at a temperature of 120 to 140°C, until 1 mole of ammonia is liberated, and
(b) cyclizing the resulting intermediate by slowly heating with a solvent at a temperature from 160 to 200°C.

According to the present invention, a simple procedure has been discovered for the preparation of macrocyclic ureas which does not involve the use of carbon disulphide or the need to prepare a diisocyanate derivative of the diamine. In addition, the procedure according to the invention does not require extremely high dilution techniques, which are often associated with the preparation of macrocyclic rings. The first step in the procedure calls for the addition of urea to the diamines under controlled reaction conditions. The first step can be carried out either in the presence, or in the absence, of a solvent. In the second step, the intermediate product (I) is slowly heated with a solvent to form the cyclicurea (II). The method can be represented by the following: 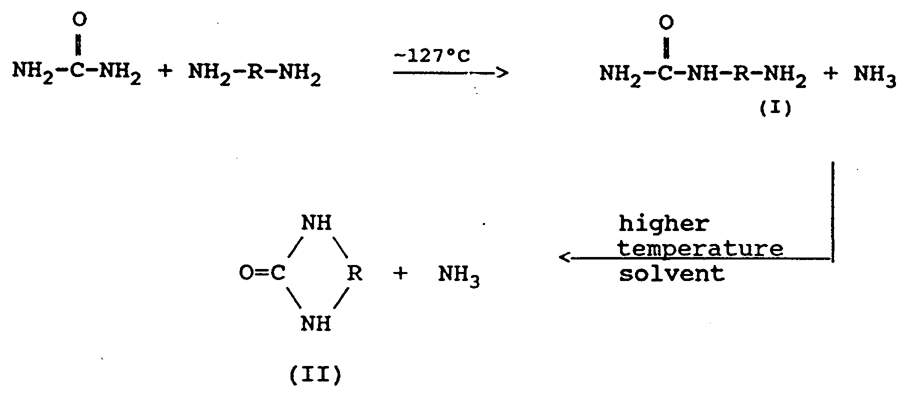
For example, R may be:

-(CH₂)ₓ-

or 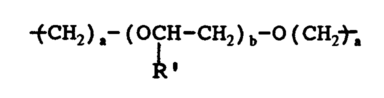
or 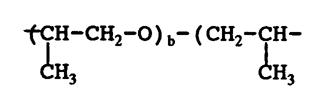
where x = 10-22, a = 2 or 3, b = 2-4 and R' = CH₃ or H.

The temperature range which is effective in the first step of the method of this invention is from 120 to 140°C. The preferred range is from 120°C to 135°C, more preferably 125 to 130°C. It is noted in Examples 1, 2, 5, 8 and 9 that ammonia evolved around 125 to 128°C. Temperatures above 140°C tend to lead to the production of linear polymers of higher molecular weight. For instance, according to Example 15 of US-A-3763106, reaction of equimolar amounts of urea and Jeffamine D-230 leads to a product with a molecular weight of 3200.

After a mole of ammonia has evolved in the first step, the intermediate (I) is reacted in a second step in a solvent, preferably a polyether or alcohol, to form the large ring (cyclic) urea (II). The intermediate (I) is added slowly to a solvent that is heated at 160 to 200°C, preferably 170 to 190°C, e.g. at about 180°C. The addition usually takes place over several hours, e.g. from 2 to 10 hours, and conveniently at or near the boiling point of the solvent, to complete the reaction.

Generally, the solvent should be an alcohol or ether. Alcohols which work include 2-ethylhexanol, dipropylene glycol, dipropylene glycol monomethylether and dipropylene glycol monoethylether. Example 6 demonstrates the usefulness of 2-ethylhexanol.

The solvent can alternatively be a polyether, including diethylene glycol diethylether and triethylene glycol dimethylether (triglyme). More preferable is the use of a methylated product of a polyether alcohol, for example, diethylene glycol dimethyl ether (diglyme) or triglyme. The Examples demonstrate the effectiveness of triglyme.

One aspect of the present invention which makes it quite desirable is that the large ring cyclic ureas can be prepared from commercially available reactants, such as long chain diamines and polyoxyalkylene diamines.

The amino groups are separated by 8 to 22 atoms, which can be carbon atoms, as in alkylene diamines, or both carbon and hetero atoms, as in polyoxyalkylene diamines.

One group of polyoxyalkylene amines, which are particularly useful in this invention, are polyethylene glycol diamines. Preferred are amine terminated polyethylene glycols having the formula: 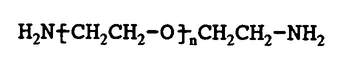
where n = 2 or 3.

This formula includes triethylene glycol diamine and tetraethylene glycol diamine. These are polyoxyethylene diamines produced by Texaco Chemical Company under the name JEFFAMINE® EDR series amines. JEFFAMINE® EDR-148 is an amine terminated triethylene glycol having the formula:

H₂N-CH₂CH₂-O-CH₂CH₂-O-CH₂CH₂-NH₂

JEFFAMINE® EDR-192 is an amine terminated tetraethylene glycol having the formula:

H₂N-CH₂CH₂-O-CH₂CH₂-O-CH₂CH₂-O-CH₂CH₂-NH₂

Other useful glycol diamines include polypropylene glycol diamines, such as tripropylene glycol diamine and tetrapropylene glycol diamine.

Also useful are other long chain diamines, such as dodecanediamine (Example 10), and polyetherdiamines with aminopropyl end groups, e.g. NH₂(CH₂)₃(OCHRCH₂)₂OCH₂CHRO(CH₂)₃NH₂ (Example 8). It is recognized that other long chain diamines with primary amine end groups and (optionally) containing functional groups that are inert under the reaction conditions may be employed.

The polyoxyalkylene diamines, such as those described above, are reacted with urea, preferably in a substantially equimolar ratio (one mole of diamine to one mole of urea). We have noted that when two moles of urea are heated with one mole of triethylene glycol diamine, ammonia begins to evolve at about 128°C. On increasing the heat to 180°C over about 8 hours period, a good yield of the bis-urea was formed: 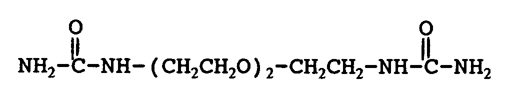
In a similar fashion, when two moles of EDR-148 were heated with one mole of urea, ammonia started to evolve at 122°C. On heating up to 140°C for 3 hours, and heating 4 hours at 160°C, a good yield of the expected diamine was obtained: 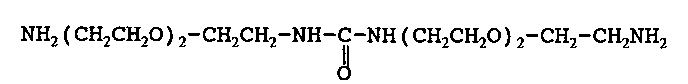
The present invention appears to proceed by means of a stepwise elimination of ammonia. Elimination of one molecule provides monocondensation products represented by: 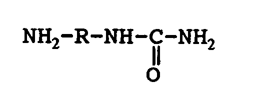
where R has, for instance, the meaning given above.

Subsequent heating in a solvent at a higher temperature within the specified range eliminates a further molecule of ammonia to provide the cyclic product. 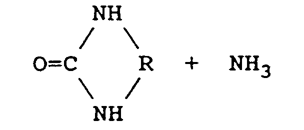
The products were identified by NMR or mass spectrometry. Although the reactions are not quantitative, they are sufficiently selective for the reaction to be used to make the ring compounds in this convenient and low cost step.

The following Examples are intended to illustrate the invention in more detail.

### EXAMPLE 1

### Tetraethylene Glycol Diamine and Urea

A 250 cm³ three-necked flask, equipped with a thermometer, stirrer and condenser, was charged with 91 grams of EDR-192 (0.5 moles) and 30 grams of urea (0.5 moles). The contents were heated to 126°C and held at 126-127°C for 3.25 hours. The product weighed 111.1 grams, and its NMR spectrum was in good agreement with the proposed structure:

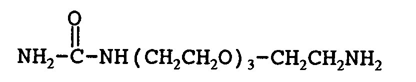

### EXAMPLE 2

### Cyclization

The product described above (25g) was mixed with 125g of triglyme and added over a three hour period to triglyme (100g) heated at 180°C.

### EXAMPLE 3

### EDR-192 and Urea

Larger amounts of the same reactants as in Example 1 were heated. A total of 384g of EDR-192 was heated with 120g of urea at 125°C for 2 hours. NMR showed that the average structure was: 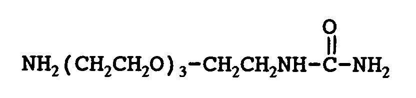
with small amounts of starting material and some bis-urea adduct. The amine content was 4.30 meq/g (theory 4.26 for the structure shown).

### EXAMPLE 4

### Cyclization

A 1000 cm³ three-necked flask (Morton), equipped with a stirrer, thermometer, nitrogen inlet and dropping funnel, was charged with 450 cm³ of 2-ethylhexanol. The alcohol was heated to 182°C and then 100g of the product described in Example 3, and 450 cm₃ of 2-ethylhexanol, were added over a 4 hour period to the alcohol kept at 182 to 184°C. Some crystals separated on cooling, and eventually a total of 49.4g of solid was collected. The product was identified as the cyclic product obtained in Example 2. Mass spectrometry indicated a molecular weight of 218.1282 which corresponds well with the desired product m.w. 218.1267 (C₉H₁₈N₂O₄).

### EXAMPLE 5

### Tetrapropylene Glycol Diamine and Urea

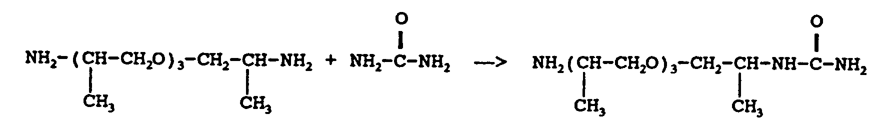

24.8g of tetrapropylene glycol diamine was heated with 6.0g of urea at 127°C to 128°C for 4 hours in a 100 cm³ flask equipped with a magnetic stirrer, thermometer, condenser and nitrogen inlet. NMR analysis indicated that about half the amine groups had reacted, and that most of the urea was monosubstituted. The product was a colourless viscous liquid. It had an amine assay of 2.84 meq/g; theory for pure product is 3.42.

### EXAMPLE 6

### Cyclization of Product from Example 5

A 500 cm³ three-necked flask, equipped with a stirrer, thermometer, addition funnel and nitrogen inlet was charged with 200 cm³ of 2-ethylhexaonol, which was heated to reflux. 19.4g of the product obtained in Example 5, in 125 cm³ of 2-ethylhexanol, was added over a 3 hour period. The contents were heated at reflux for an additional 2 hours and the solvent removed by distillation to obtain 20.4g of material. Mass spectroscopic analysis showed a small amount of cyclic product with a m.w. of 274.2163 (actual m.w. of the macrocyclic is 274.1893). IR analysis indicated a mixture of products.

### EXAMPLE 7 (Comparison)

### Bis(aminoethyl) Ether and Urea

Attempts to form a cyclic product from bis(aminoethyl) ether and urea were unsuccessful. Polymeric materials were formed. It is known that compounds having 7 and 8 membered rings polymerize readily with heat alone.

### EXAMPLE 8

### Diethylene Glycol Bis(propylamine) and Urea

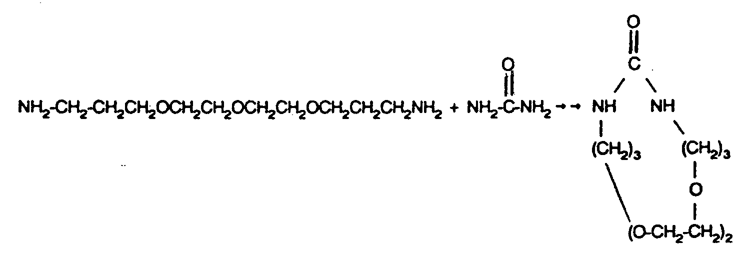

The amine (44g) was heated with 12g of urea at 124 to 127°C for 3 hours, giving 52g of product. This product, 25g, dissolved in 150 cm³ of 2-ethylhexanol, was added to 200 cm³ of boiling 2-ethylhexanol. (material had to be removed from the addition funnel to keep a homogenous solution). Most of the solvent was removed, and the product was washed with isopropanol. The product was difficult to filter, but after getting a wet cake, the solvent was removed at 70°C and 40 Pa. The product weighed 12.2g. Mass spectrometry showed that the monomeric cyclic product was found in good yield. The calculated m.w. was 246.15996; the actual m.w. was 246.15796.

### EXAMPLE 9

### Triethylene Glycol Diamine (EDR-148) and Urea

269g of EDR-148 were allowed to react with 120g of urea. The urea was added to EDR-148 and heated to 80°C over a 40 minute period. The mixture was heated to 126°C and held at 126 to 127°C for 2 hours. NMR showed that about 17% of the product was disubstituted urea. 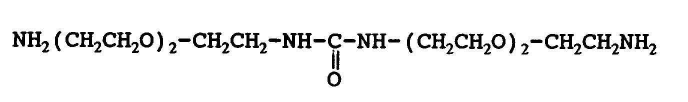
Nevertheless, 100g of this material in 2-ethylhexanol was added to 450 cm³ of boiling 2-ethylhexanol over a 5 hour period. Crystals separated when the solution was cooled. A total of 92.2g of crystals was obtained, after filtering and washing twice with 250 cm³ of cyclohexane. The crystals were heated with isopropanol and dried to give 77.5g of material, melting at 132 to 148°C with some changes occurring below 132°C. Mass spectrometry indicated that some of the material was the expected cyclic product: a m.w. of 174.1008 was detected, which corresponds well with the desired product m.w. 174.1004. Attempts to sublime this material resulted in polymerization. This was not unexpected, since Makacyama et al., J. Am. Chem. Soc. 79, 4358, (1957) indicated that cyclic ureas, especially those with 7 to 10 members in the ring, were thermally unstable.

### EXAMPLE 10

### Dodecane Diamine and Urea

A 1000 cm³ three-necked flask, equipped with a stirrer, thermometer, nitrogen inlet, and condenser, was charged with 200g of dodecane diamine, 400 cm³ of 2-ethylhexanol and 60g of urea. The contents were heated at 127°C and held at this temperature for 3 hours. On cooling, the material settled as a homogenous white cake. An additional 300 cm³ of 2-ethylhexanol was added and the mass was heated to 105°C. Portions of this material were added to boiling 2-ethylhexanol over a five hour period. The contents were heated at 185°C for an additional four hours, cooled to 50°C and the solid was filtered and dried. The cyclic diamine urea C₁₃H₂₆N₂O was obtained (m.w. 226). It was contaminated with a little amine (0.085 meq/g). 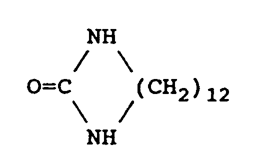
The product melted at 207° to 213°C.

## Claims

1. A method for preparation of cyclic ureas characterised by:
(a) heating urea with a diamine whose amino groups are separated by 8 to 22 atoms in a molar ratio of about 1:1, at a temperature of 120 to 140°C, until 1 mole of ammonia is liberated, and
(b) cyclizing the resulting intermediate by slowly heating with a solvent at a temperature from 160 to 200°C.

2. A method according to Claim 1 characterised in that the diamine is a polyoxyalkylene diamine having the formula: 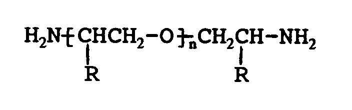 wherein R = H or CH₃ and n = 2, 3, 4 or 5.

3. A method according to Claim 2 characterised in that the polyoxyalkylene diamine is a polyethylene glycol diamine having the formula: 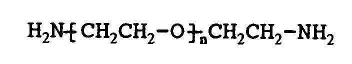 wherein n = 2, 3 or 4.

4. A method according to Claim 2 characterised in that the diamine is tetrapropylene glycol diamine or tripropylene glycol diamine.

5. A method according to Claim 2 characterised in that the polyoxyalkylene diamine is a polyoxyalkylene bis(propylamine) with the formula:
NH₂-CH₂CH₂CH₂(OCH₂CH₂)ₙOCH₂CH₂CH₂NH₂
wherein n = 2 or 3.

6. A method according to Claim 1 characterised in that the diamine is dodecane diamine.

7. A method according to any one of Claims 1 to 6 characterised in that the solvent is an alcohol or polyether.

8. A method according to Claim 7 characterised in that the solvent is triethylene glycol dimethyl ether (triglyme).

9. A method according to Claim 7 characterised in that the solvent is 2-ethylhexanol.

10. A large ring cyclic urea having the formula: 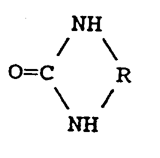 wherein R is: 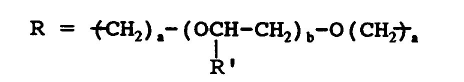 or 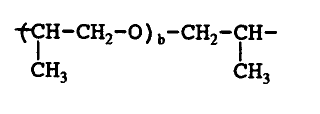 a = 2 or 3, b = 2 to 4, and R' = CH₃ or H.
